# EUROPEAN PATENT SPECIFICATION

(11) **EP 1 971 300 B1**
(45) Date of publication and mention of the grant of the patent: **19.08.2015**
(21) Application number: 07701226.8
(22) Date of filing: 11.01.2007
(51) Int. Cl.: A61F 5/44, A61F 5/445

(54) **COLOSTOMY BAG CLEANING SYSTEM**
REINIGUNGSSYSTEM FÜR KOLOSTOMIEBEUTEL
SYSTÈME DE NETTOYAGE POUR POCHE DE COLOSTOMIE

(30) Priority: 11.01.2006 US 328599
(43) Date of publication of application: 24.09.2008
(73) Proprietor: Schena, Kenneth R., Naples FL 34105-2568 (US); Schena, Blaine M., Naples FL 34105 (US)
(72) Inventor: Schena, Kenneth R., Naples FL 34105-2568 (US); Schena, Blaine M., Naples FL 34105 (US)
(74) Representative: Neobard, William John
(86) International application number: PCT/US2007/060397
(87) International publication number: WO 2007/082270

(56) References cited:
- WO-A1-2005/063152
- WO-A1-2005/063152
- DE-U1- 8 706 956
- US-A- 3 902 496
- US-A- 4 654 037
- US-A1- 2003 139 271
- US-A1- 2003 220 621
- US-A1- 2003 220 621
- US-A1- 2005 075 616
- US-A1- 2005 283 126
- US-A1- 2005 283 126
- US-B1- 6 224 581
- US-B1- 6 224 581
- US-B2- 6 685 648

## Description

### BACKGROUND OF THE INVENTION

A colostomy is a surgical procedure in which the colon or a portion thereof is removed and the digestive tract is attached to an opening created in the abdominal wall, thereby, allowing digested waste to pass through the abdomen. Typically, the waste is then collected by an impervious bag that is secured over the opening. The opening that results from a colonectomy is know as an "ostomy' or a "stoma," and the impervious bag that collects the digestive waste is known as a colostomy bag.

An individual who has had a colostomy must typically remove and empty the colostomy bag several times a day, and must irrigate the ostomy at least every other day to maintain good health and sanitation. An ostomy is irrigated by applying flowing water into the ostomy and then allowing the water to drain.

Examples of ostomy irrigating devices in the prior art or cleansing systems are provided by U.S. Patent Application Publication No. US2003/0229324, which features a closed drainage system that eliminates the necessity to stand over a toilet, as it has its own collection system, but it is a difficult system to use and almost requires the person to be lying down as illustrated in Figure 1 for any satisfactory use. U.S. Patent No. 6,408,861 illustrates a urine bag cleaning manifold, which is a very complicated system associated with a shower in a bathtub, and again, is difficult to operate and very elaborate in design. Patent No. 5,454,389 teaches an ostomy bag cleaning device that incorporates a mechanism for introducing water into a colostomy bag and then evacuate the waste material into a storage chamber. This device is cumbersome to use and does not provide for a simple cleaning of the colostomy bag without removal form the person.

Patent Nos. 5,096,503 and 4,194,506 both teach the general concept of insertion rods being inserted from the bottom of the colostomy bag up into the bag itself, and in both of these systems it's awkward to have to insert a rod up through the bottom of the colostomy bag with whatever drippings and materials that would be coming out, and this is not a satisfactory technique for cleaning the colostomy bag while having the bag still maintained on the person. A similar patent is 5,738,668, which again inserts a probe up into the bag for cleaning, and again the same problems are inherent. Patent No. 6,532,971 teaches a sanitary pouch washer that is designed for simultaneously cleaning the inside and outside of the colostomy bag and is a complicated mechanism and, again, is done with the colostomy bag removed from the person. U.S. Patent No. 6,224,581 teaches a colostomy bag cleaning appliance having a mounting plate and, again, this is a cleaning method with the bag removed form the person and creates significant complications in achieving the cleaning in a simple and effective manner.

Patent No. US 4,654,037 discloses an ostomy pouch having a self contained irrigator for cleaning the pouch. The pouch comprises spaced front and back walls sealed about their peripherals to define a collecting chamber and has a closable bottom opening for draining the collected contents of the pouch for disposal. An opening is provided in one of the walls for receiving the stoma of a patient. A fluid distribution tube is located within the pouch and is provided with a multiplicity of openings so that a projecting end of the tube may be connected to a source of cleansing fluid to permit the contents of the pouch to be flushed from the pouch without removing the pouch from the patient's body.

Patent Publication No. US 2005/0283126 discloses a colostomy bag cleaning system. The disclosure relates to a closed drainage system for irrigating colostomy bags wherein the bag is irrigated and cleaned while in its operable position associated with the person. More specifically, a manifold is mounted within the colostomy bag with an access to a water pressure from outside, the manifold providing a spray dispersion of fluid, or water preferably, at the top of the bag and gravity then drips it down through the bag and out the open bottom, preferably with the residue of the bag being washed out by the water and passing into a toilet where the user flushes the bag to completion of cleaning with all residue from the bag passing into the toilet. A water source from the toilet itself, or other suitable water supply, provides the pressured water to the manifold inside the top of the colostomy bag. This system allows for a large volume colostomy bag irrigation and eliminates messy bag cleaning by removal from the person, the possibility of spillage, odors, and the spreading of infection diseases.

Patent Publication No. US 2005/0075616 discloses ostomy tools and systems and processes for their use. Waste management for people with an ostomy (ostomists), and who therefore find it necessary to wear an ostomy bag, is accomplished simply through processes, systems and ostomy tools involving versatile latent tubes. Such tools include ostomy bags, filters, connectors and conveyance vessels. The tools are particularly helpful, for example, in controlling the location and disposition of ostomy wastes (and their odors) when the ostomist performs challenging routine to unusual tasks associated with ostomy care and maintenance. The tools also help to instill confidence in the users' ability to carry on normal daily business and social activities without creating embarrassment or discomfort for themselves or others.

### SUMMARY OF THE INVENTION

In accordance with the invention, there is provided a colostomy bag cleansing system as defined in accompanying claim 1. Preferred embodiments are defined in the dependent claims. A method of cleaning the colostomy bag of a cleansing system according to the invention is also provided in independent claim 10.

A closed draining system is described for externally cleansing ostomies and cleaning colostomy bags utilizing a manifold physically located near the top of the bag that provides a stream of water in a sprayed fashion into the top of the bag for internally cleaning the bag and simultaneously cleaning the exposed surfaces of the ostomy, and with the amount of water or cleaning solution added being controlled by the user, the bag still being in place on the person, and the flow from the bag coming out the bottom with the normal opening type bags available today. The cleansing system is an integral part of the pouch.

It is an aim of the invention to facilitate cleaning of the colostomy bag and simultaneously cleaning the exposed surfaces of the ostomy by providing a manifold near the top of the colostomy bag that is connected to a source of water under some pressure that can then controllably sprinkle and/or spray water into the colostomy bag for cleaning of the bag and the ostomy, with the flow then directed out the bottom of the normal opening-type colostomy bags. During cleansing, the person still has the colostomy bag attached to their body, and the cleansing takes place in association with drainage into a toilet or other suitable drainage facility. This system may be an integral part of the pouch to allow convenient use at the discretion of the user by simply applying water under pressure to the inlet port while sitting on a commode to receive dispelled waste.

Embodiments of the invention provide the ability for cleaning of the colostomy bag with the bag still attached to the body of the person, and it can be done quicldy and very effectively on a regular basis during the day or any desired time or times.

Embodiments of the invention provide improved elements and arrangement for the purposes described, and yet are inexpensive to manufacture, dependable and fully effective in accomplishing that intended purposes.

These and other aspects of the present invention will become readily apparent upon further review of the following specifications and drawings.

### BRIEF DESCRIPTION OF THE DRAWINGS

Figure 1 is a perspective view of a colostomy bag, not covered by the present invention, utilizing a manifold around the flanged opening on the bag itself, which then attaches to the flanged fitting positioned on the body of the person, and snaps together to form a fluid-tight seal between the bag and the person;
Figure 2 is a perspective illustration showing irrigating and flushing the colostomy bag by controlling a valve from the water supply to the toilet with the bottom of the colostomy bag open and flow taking place from the manifold through the bag and into the toilet;
Figure 3 is a perspective illustration of a known manifold associated with the flanged ring and adhesive backing for attachment to a person, for engaging in a fluid-tight relationship with the fitting on the colostomy bag, with the colostomy bag itself removed from the Figure to depict the mated relationship between the flanged ring attached to the person and the fitting on the colostomy bag;
Figure 4 illustrates a known colostomy bag with the manifold positioned around the top of the formed snap connection for the bag itself and water inlet being provided to the manifold around the top of the bag connection opening;
Figure 5 is a cross-sectional illustration of a colostomy bag according to the present invention, having a manifold heat-seal formed into the material itself and at the very top access opening into the bag well above the stoma opening;
Figure 6 is a top-plan view of a colostomy bag showing some hook and loop strips along the bag, to be folded up in half and held in that position;
Figure 7 is the bag shown in Figure 6 folded-up, reduced in half its length in the position where the bag is held in the folded position by the hook and loop strips;
Figure 8 is a cross-sectional illustration of the bag of Figure 5 taken on line 8-8 of Figure 5;
Figure 9 is a broken-away cross-sectional view of the bag of Figure 5 taken on line 9-9 of Figure 5;
Figure 10 is a plan view of an embodiment of a colostomy bag according to the present invention, having a cleansing system integrally formed therein as an upper manifold formed into the material itself such as by dot welds, adjacent the very top access opening of the bag, with a modulated array turbulence producing structures and openings arranged throughout the integral manifold;
Figure 11 is a plan view of a further embodiment of a colostomy bag, having a manifold integrally formed into the material itself adjacent a top access opening of the bag; and
Figure 12 is a perspective view of a colostomy bag and cleaning system for cleaning of the colostomy bag.

### DESCRIPTION OF THE INVENTION

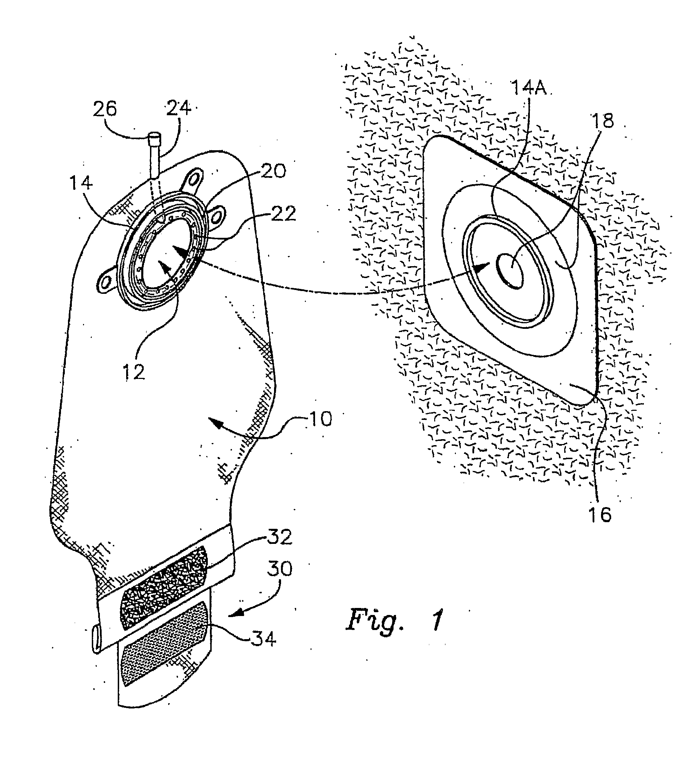

Figures 1 to 4 relate to a known colostomy bag and system.

Referring now to Figure 1 of the drawings, the numeral 10 indicates a colostomy bag, which has an opening 12 that is surrounded by a plastic ring 14 and attached in a fluid-tight fashion to the bag 10 by suitable means, such as adhesively or by other suitable known means. The ring 14 is formed with a flange that is designed to mate with a similarly formed ring and flange 14A on the person mounted colostomy bag attachment patch 16. The patch 16 is attached by pressure-sensitive adhesive on the back of the patch itself and the respective ring flanges 14 and 14A when snapped together form a fluid-tight seal between the ostomy 18 and the interior of the colostomy bag 10.

A cleansing system is provided by a circular manifold, generally indicated by numeral 20, which is preferably a soft plastic and encircles the interior of the opening 12 into the colostomy bag 10. A plurality of small holes 22 around the perimeter of the manifold 20 are provided to allow the passage of spray in multiple and random directions of water pressure introduced through an access tube 24 that is connected to a water source not shown via fitting 26. Alternatively, the manifold 20 may be integrally formed around opening 12, such as by heat-sealing or the like.

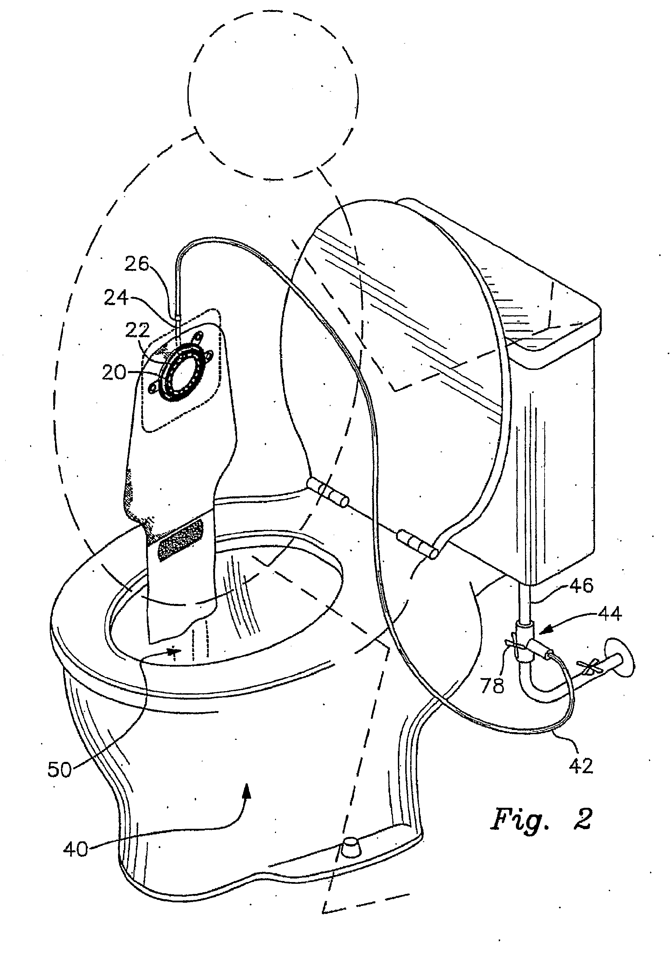

The colostomy bag 10 is designed to be opened at the bottom end, generally identified by numeral 30. For use, this bottom opening is rolled up and retained in a position to close the opening, such as by a sealing procedure utilizing hook and loop material indicated by the loop material 32 and the hook material 34. It should be understood that when this bottom end 30 is folded up and the hook and loop attachment is in place, the bag is sealed at the bottom. For selective cleansing of the bag and exposed portion of the ostomy, the bag is opened and positioned over the toilet so that irrigation cleansing water or solution passing through the manifold 20 and orifices 22 will flow down through the bag and out the bottom opening 30 and into the toilet or the like, as is shown in more detail in Figure 2 of the drawings.

Referring now to Figure 2, this illustrates a normal toilet indicated generally by numeral 40, on which the individual will normally sit toward the back on the toilet seat to operate the invention. A flexible water or supply hose 42 is selectively connected to the fitting 26 to provide water under pressure through the conduit 24 and to the manifold 20 for spray out the orifices 22. The water source is by a fitting indicated generally by numeral 44 that fits into the normal water supply tubing to the toilet, indicated by numeral 46, and has the ability to control the amount of flow by a valve, indicated by numeral 48, associated with the conduit 42.

Thus, with reference to Figure 2, it can be understood that the individual sits on the toilet seat, connects the conduit 42 to fitting 26 and then, by adjustively controlling the valve 48, provides a sufficient amount of water under pressure into the manifold to cause a flow to cleanse the ostomy 18, and to provide a cleansing action within the colostomy bag 10, with the residue dripping out at the bottom into the toilet as shown generally by numeral 50.

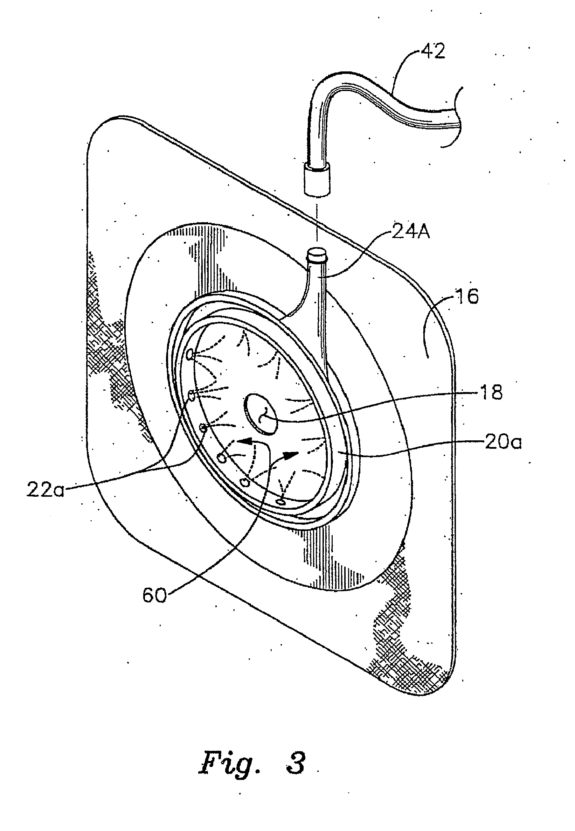

Referring now to Figure 3, this shows an embodiment where the manifold, identified generally as numeral 20a is associated with the ring 14A of the patch 16 that is attached to the skin of the person over the ostomy opening 18. In this embodiment, the manifold 20a includes an access fitting 24A selectively connected to the water line 42 so that regulated water under pressure provides the spray through orifices 22a illustrated generally by the numeral 60.

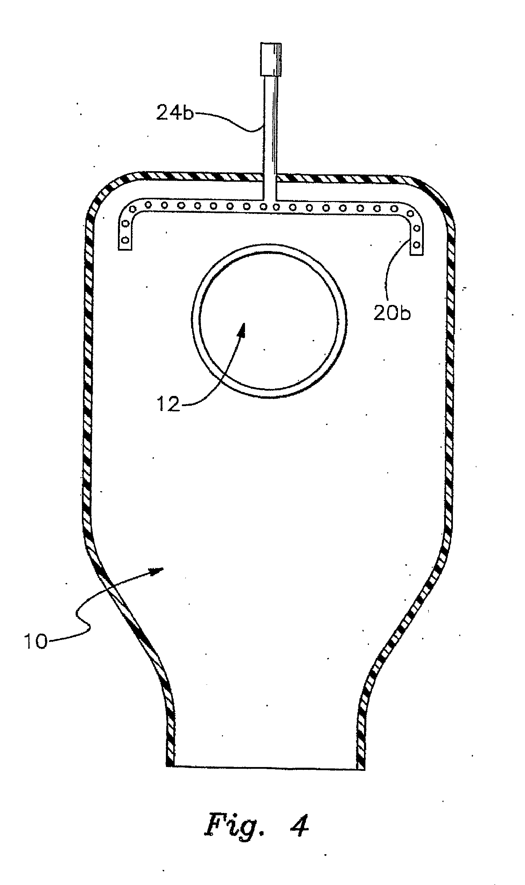

Referring now to Figure 4, this shows an embodiment where the manifold, indicated generally by numeral 20b, is positioned above the opening 12 in the colostomy bag 10. A similar water inlet or access fitting 24b may be utilized in this embodiment. In this embodiment, the manifold 20b may be positioned adjacent the top of bag 10 to disperse water or cleaning fluid under pressure throughout the bag 10 for quick and effective cleaning of bag 10. The spray from the plural outlets formed in manifold 20b also will flow over and around exposed portions of the ostomy for cleansing thereof. The upper manifold 20b may be used alone or with a manifold positioned about the ostomy opening if desired.

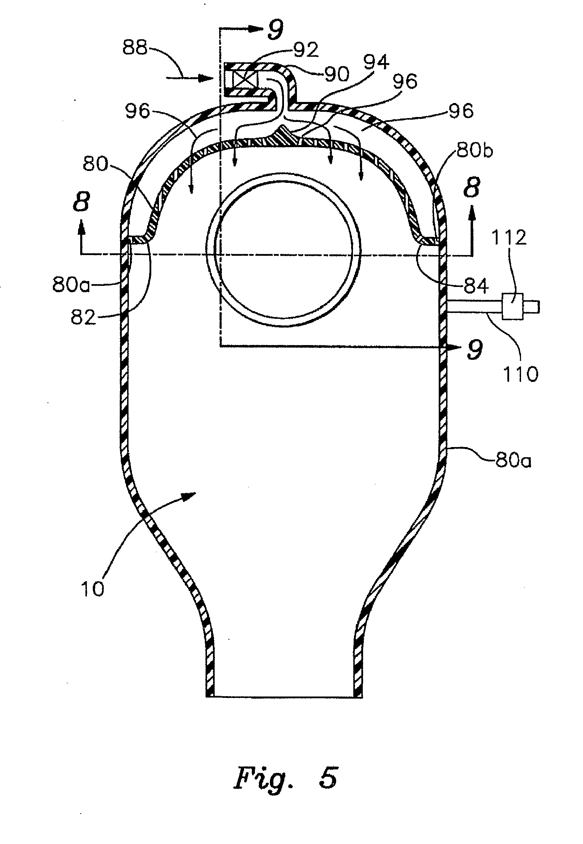

An embodiment comprising an upper manifold according to the present invention is shown in Figure 5. More particularly, the manifold in Figure 5 is integrally formed in bag 10 by heat-sealing the bag 10 along the line shown generally by numeral 80, which may extend from the edge of the bag as shown on the left side at 82 and around below the bottom of the top of the bag and over to the right side, indicated by numeral 84. The sealed line 80 may mirror the shape of the top edge of the bag to form a uniformly dimensioned manifold across the entire top of the bag 10. The plurality of openings 86 are provided through the heat-sealed portion 80, and this then provides for the random and multi-directed flow of water sprayed from the manifold after being supplied under pressure into the manifold from the supply opening in the direction of arrow 88. The access opening into the bag is indicated generally by numeral 90, is integrally formed in the bag 10, and may include a one-way valve 92 that prevents back-flow up through the opening 90 in case the bag is squeezed or actuated through the force of fluid back up through the opening 90 in the bag. There may also be a divider indicated generally by numeral 94 that is part of the heat-formed portion of the manifold, which acts to direct the water flow entering through opening 90 in opposite directions in the manifold around to the right and the left sides of the bag as is indicated by the water flow arrows indicated generally by numeral 96.

Thus, it should be understood in the embodiment of Figure 5 that the water flows in the direction indicated by arrow 88 through the one-way valve 92 and into the manifold formed by the heat-sealed line 80 and is directed substantially equally right and left by the divider 94 and then flows through the multiple openings 86 formed in the manifold, as indicated by the water flow by numeral 96. The plurality of openings 86 provide for dispersement of water or cleansing solution from the manifold throughout the bag 10, forming sheets and sprays of water in random directions from the very top of the colostomy bag including down over the stoma opening and then as it drains out through the bottom opening as described previously. Also note the openings 86a and 86b immediately adjacent the outer edge of the bag that send a water spray down the inside edges of the bag for better cleaning of these surfaces.

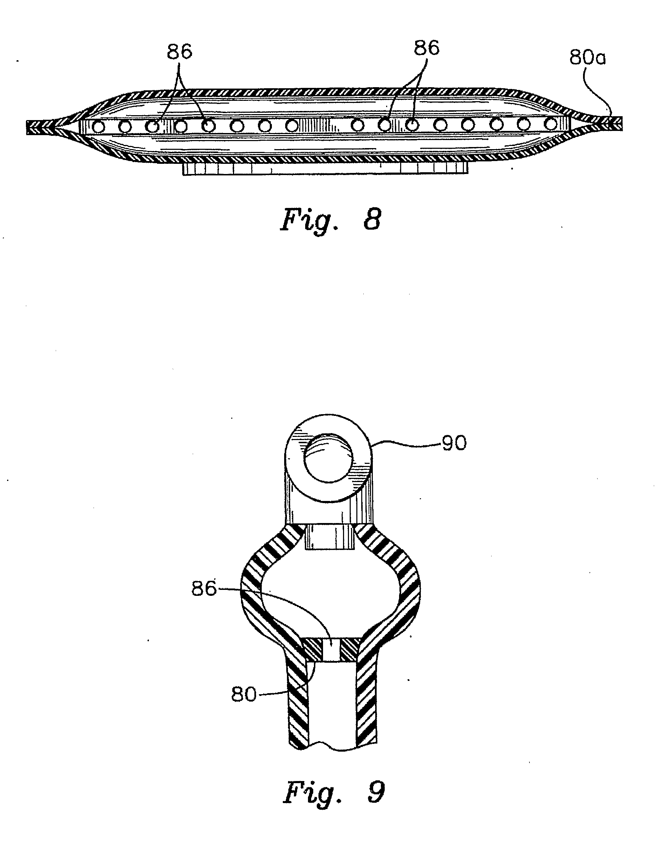

Thus, it is seen in Figure 5 and as also shown in the cross-sectional configurations of Figures 8 and 9 that the manifold is enhanced and enlarged, while being easily manufactured integral to bag 10 by the heat-sealed line 80 adjacent the top of the bag. The random directed openings 86 provide for quick and efficient cleaning of the entire bag 10. It should be understood that the bag 10 can be formed from two similarly shaped essentially flat flexible pieces of polymer material that are heat sealed at the periphery in a heat-sealed line 80a as seen in Figures 5 and 8.

Figure 9 illustrates the cross-sectional portion of the bag of Figure 5 taken on line 9-9 and it is depicted in the mode where there is water under pressure within the manifold to form a tubular configuration in the manifold itself as it is pressurized by the water entering in the direction of arrow 88 as shown in Figure 5.

Figure 8 shows the multiple openings 86 that are formed by the heat-seal across the manifold-forming heat-seal line 80, as shown in Figure 5.

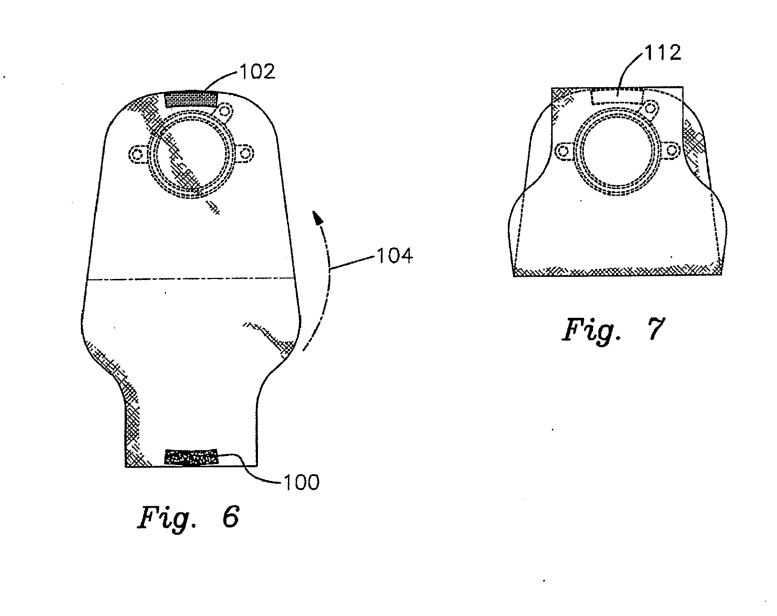

Figures 6 and 7 shown hook and loop attachments 100 and 102, which allow the bag 10 to be folded from the position shown in Figure 6 as indicated by the arrow 104 to the half-position shown in Figure 7. This is convenient in certain times when the bag is not really full to allow a person to have a smaller bag than the full length bag of Figure 6, and it greatly facilitates keeping the bag from interrupting sleeping because of the smaller size.

Thus, it should be understood that the bag and cleansing system according to the invention is very simply and cost effectively made, with the embodiments using an integrated manifold or manifolds further enhancing these attributes. In the embodiment of Figure 5, the heat-seal line 80 may be formed in the bag according to standard heat-seal procedures while simultaneously forming the openings 86 so that there will be a random and plentiful flow, much like a shower, from the openings and the water under pressure being applied through the supply opening 90 into the top of the manifold formed by the heat-sealed line 80.

Thus, it should be fully understood that Figures 5, 8 and 9 show the heat-sealed pouch configuration consisting of two chambers separated by a heat-sealed septum, line 80, containing a number of communication ports 86 connecting the two chambers. The upper chamber (or plenum) being the smaller of the two, with the system for being connected to an external water supply also integrated therewith. The lower chamber is the larger of the two, with the system for opening the bottom of the bag to accommodate the removal of its contents during cleansing. The communication ports 86 connecting the two chambers are strategically positioned to distribute the water that enters through the smaller, or upper, chamber in a manner that thoroughly cleanses the complete interior of the larger (or lower) chamber. It will also thoroughly cleanse around the stoma opening at the same time including exposed surfaces of the stoma.

The heat seal of the tube to the film material and the outer perimeter and septum geometry may be done using RF (Radio Frequency or dielectric) sealing methods. Impulse sealing can be used for the outer perimeter and septum seals, but any suitable method is contemplated. In an RF sealing method, the actual sealing takes place by locating the layers of the film material onto a fixture that is called a receiver. The sealing die, which is machined to the exact dimensions of the seal design, is then lowered onto the top face of the film layers under a required pressure, and the RF is actuated, which generates an instant heat at the interface of the upper and lower film materials. This creates the desired seal and openings 86 as an example.

The ports 86 are created by machining slots through the heat sealing die in the defined locations. Because the heat sealing process required two flat surfaces pushing together under a pressure to create the seal, these slots create interruptions in the flat surface, and therefore are not pushing the material together at those locations. Because the material does not have intimate contact at those points, it will not seal together, therefore creating the holes through the septum.

In the embodiment of Figure 5, the manifold has 18 passages which are approximately 0.229cm (.090 inches) wide when the material is lying in the flat position. When the water distribution chamber (or plenum) is filled with the pressurized water, the passage than change their geometry to a rounder hole with a diameter that approaches 0.152cm (.06 inches) in diameter. Their actual shape is probably more oblong, but the effective opening is that of a round hole with that cross section. The size of openings 86 may vary as desired, for example, between 0.127 and 0.305cms (.05 to .12 inches) diameter. The total number of openings 86 could also vary from about 14 to 24.

It is further noted that the system is pressurized by water under pressure coming from a water line that normally provides water to the toilet itself or something similar thereto. As is also shown in Figure 5, there may be provided in association with bag 10 an external port 110 from the plenum to vent accumulated gas through a charcoal filter 112, thus allowing gas relief without odor.

It should be understood that while Figure 2 illustrates the water under pressure as coming from a water line 46 that normally provides water to the toilet itself, the invention contemplates that any suitable source of water or cleansing solution under some pressure for supply to the one or more manifolds according to the invention. For example, a bottle of water with the ability to squeeze the bottle may provide suitable fluid under pressure. Similarly, a small battery-operated pump with a water source may provide the desired fluid under pressure. It is believed that the invention will best be set up for a person to utilize in their own bathroom in their own home. However, there may be instances when they are traveling or not provided with a facility set up with the ability to hook the water hose to the adaptor 26 and feed the conduit 24 into the manifold, and thus any source of cleansing solution under pressure may be suitable.

It is also to be understood that the spray holes 22 in the manifold in Figure 1 are of no particular consequence except to provide a suitable, fairly fine, spray that will tend to both wash and clean and irrigate the residue inside the colostomy bag itself so as to facilitate cleaning in the shortest possible time. It has been found that this system can provided cleaning in just a few minutes or less, and can be done several times a day very conveniently by virtue of the very simple application of water under pressure through the manifold to clean the colostomy bag itself.

It is to be understood that the provision of a manifold that is positioned high in the colostomy bag, and which provides a large spray pattern clears the bag from the top down, and it has been found it is desirable to have the manifold positioned no lower than the opening 12 in the colostomy bag for attachment to the stoma flange 14A. This then provides for good gravity flow of the water being used in the cleansing system, allowing it to flow down through the bag, picking up all residue and dispensing it out the bottom opening 30.

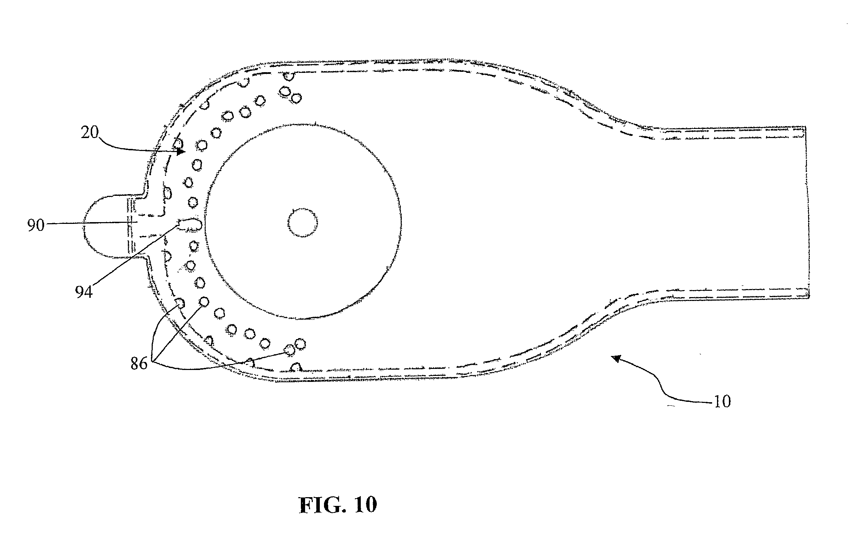

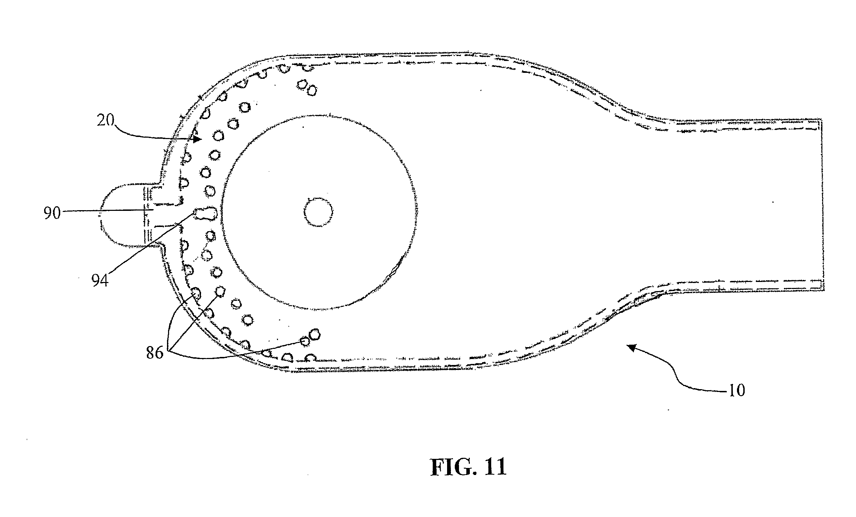

Referring now to Figures 10 and 11, alternate embodiments of the invention are shown with modifications made to the integral manifold construction according to the invention. More specifically, in the embodiments of Figs. 10 and 11, the manifold 20 is integrally formed in the topmost portion of the colostomy pouch or bag 10, with the top wall of the pouch forming the top of the manifold 20. The bottom of the manifold 20 is provided by a series of dot welds 86, which may be referred to as bounce dot welds due to the function thereof during the cleaning operation. The dot welds 86 together define the bottom of the manifold 20, causing restriction of the flow of water or cleaning solution from the inlet 90, so as to distribute the cleaning solution throughout the manifold 20, and thereby throughout the entire pouch 10 as desired for effective cleaning of the entire pouch 10. The dot welds 86 also define openings therebetween which the cleaning solution will be distributed through for cleaning of the entire pouch 10. It should be understood that the dimensions of the dot welds 86 may be altered to provide the desired flow characteristics, with smaller diameter welds 86 providing additional or increased flow characteristics, and larger diameter welds 86 tending to reduce flow characteristics. Further, the spacing between the dot welds 86 can be modified to provide desired flow characteristics through the spaces between welds 86.

Additionally, as seen in the embodiments of Figs. 10 and 11, the spot welds 86 forming the bottom of the manifold 20 may be staggered relative to adjacent welds 86 to facilitate causing turbulence in the cleaning solution as it exits the manifold 20 to be effectively dispersed throughout the pouch 20. The staggered arrangement of the welds 86 may also be modified to create desired turbulence and flow characteristics from the manifold 20. The turbulence created by the lower staggered welds 86 also facilitates distribution of cleaning fluid to the stoma and stoma opening of the pouch 10 for effective cleaning of the stoma and area surrounding the stoma opening. To further facilitate desired distribution of cleaning fluid in the pouch 10, spot or line welds 86 may be formed to create a larger area of restriction to flow of the cleaning fluid, and as shown in these embodiments, such areas may be formed adjacent the inlet 90 at 94, to cause dispersion of the flow of cleaning solution away from the center portion of the pouch 10. Further, such enlarged areas of restriction may be formed at the lower outside portions of the manifold 20, to somewhat restrict flow at these locations to facilitate dispersion of cleaning solution throughout the manifold 20. In addition, spot welds 86 may be formed on the upper portion of the manifold 20 to facilitate creating turbulence in the cleaning fluid as it enters and exits the manifold 20. In the embodiments of Figs. 10 and 11, the number of upper spot welds 86 in the manifold 20 are different to provide alternative affects of creating turbulence. As it should be understood, as cleaning solution is introduced into the inlet 90, and into the manifold 20, the solution will be effectively dispersed throughout the manifold 20, and will be turbulently released from the manifold to be dispersed throughout the pouch 10 in a turbulent fashion to clean the pouch 10 completely, as well as to disperse turbulent solution across the stoma and area surrounding the stoma opening for effective cleaning thereof. As also seen in the embodiment of Fig. 11, there may be increased openings between welds 86 adjacent the outside ends of the manifold 20 to facilitate distribution of cleaning solution at these locations. Various modifications of the manifold configuration are contemplated, such as a different configuration of welds other than spot welds, the dimensions or relative configuration of welds or the like. The welds 86 forming and in the manifold 20 may be arranged in a modulated fashion to direct the water flow throughout the colostomy bag 10 and to create turbulence, to enhance cleansing of the bag. The modular arrangement of openings 86 directs water down throughout the colostomy bag 10 at the side walls of the bag and at the stoma opening, while providing a cost effective manufacturing approach in forming the manifold 20 and its characteristics.

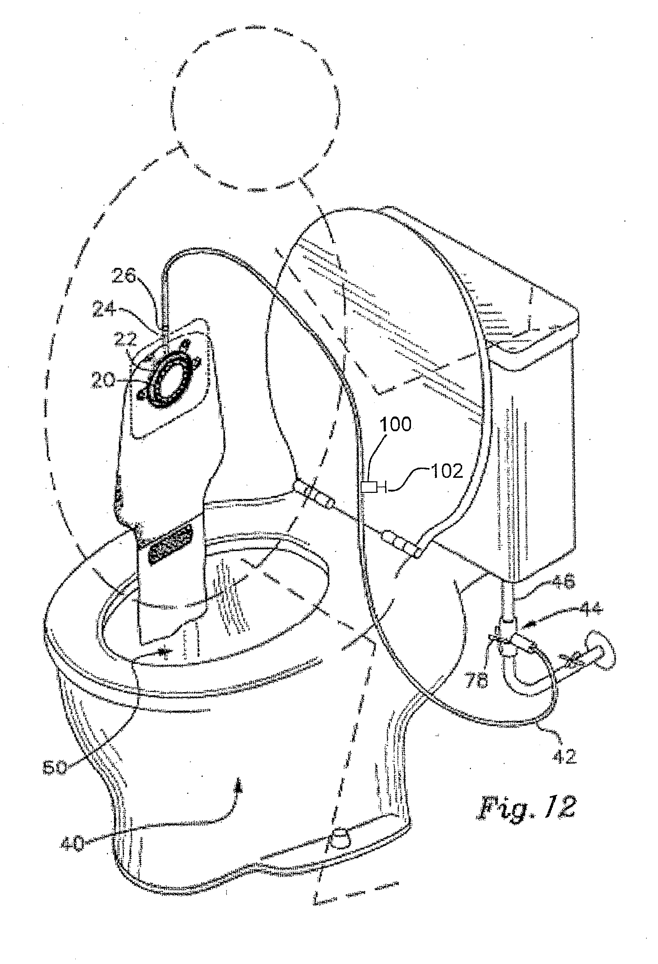

As shown in Fig. 12, there may be provided a small sump 100 formed in the feed line 42, having an actuator valve or button 102, which can be selectively used to dispense a small amount of a cleaning, deodorizing, disinfecting, acid neutralizing and/or lubricating materials into the feed line 42 for introduction into the pouch 10. The introduction of such materials can facilitate cleaning and/or deodorizing of the pouch, and/or use by facilitating both the cleaning, disinfecting and/or moisturizing or otherwise treating the stoma while the pouch is attached and in use on a patient. The system for accomplishing this may also be a separate system if desired, which could be selectively attached to the inlet line 42. Various modifications to achieve similar functions are also contemplated.

It is to be understood that the scope of the invention is not to be limited by the descriptions and explanations set forth above, but that the invention encompasses any and all embodiments within the scope of the following claims.

## Claims

1. A colostomy bag cleansing system comprising:
a colostomy bag (10) having a bottom drain opening (30), a stoma opening (12), and a water supply opening (90) at the top of the bag, said bag being formed of two similarly shaped sides that are held together by a first heat-sealed line (80a) around and adjacent to the perimeter of the sides to form the bag,
a heat-sealed septum (80) comprising a plurality of second heat-sealed portions formed between the sides along and spaced from the top periphery of the bag to divide the bag into an upper manifold (20) and a lower chamber, with the stoma opening and bottom drain opening provided in the lower chamber, and the supply opening connecting into the upper manifold, the plurality of heat sealed portions defining a plurality of passages (86, 86a, 86b) to connect between the upper manifold and lower chamber, and a system to provide a fluid under pressure to the supply opening and into the manifold,
the second heat-sealed portions being configured such that fluid is sprayed in multiple directions through the plurality of passages from the upper manifold to the lower chamber when fluid under pressure is provided to the supply opening.

2. A system (10) according to Claim 1, said manifold (20) being formed by a heat-sealed line (80) spaced from the top edge of the bag and having a plurality of holes (86) formed in the heat-sealed line (80).

3. A system (10) according to Claim 1, said second heat-sealed portions being formed by dot welds (86).

4. A system (10) according to Claim 3, wherein
the plurality of dot welds (86) are staggered in their spacing from the top edge of the bag (10), or
wherein the plurality of dot welds (86) comprise dot welds positioned adjacent the top edge of the bag (10), or
wherein the plurality of dot welds (86) comprise dot welds adjacent one another, or
wherein the diameter of the dot welds (86) is variable.

5. A system (10) according to Claim 1, further comprising a sump (100) provided in the system to provide fluid under pressure with an actuator (102) to selectively introduce a treatment material into the fluid under pressure.

6. A system (10) according to Claim 1, wherein said manifold (20) contains a divider (94) adapted to direct fluid flow away from the center portion of the manifold (20).

7. A system (10) according to Claim 1, which includes means for venting accumulated gas through an odor-controlling filter (112).

8. A system (10) according to Claim 4, wherein the passages are between 14 and 24 in number and between 0.127 and 0.305 cm (.05 and .12 inches) respectively, in dimension.

9. A system (10) according to Claim 5, which includes a securing system (100, 102) to fold the bag (10) on itself and hold the bag (10) in a position where it is approximately half the length of the full length bag.

10. A method of cleaning the colostomy bag (10) of a cleansing system according to any of claims 1 to 9 comprising the steps of:
selectively connecting a fluid under pressure through the supply system to the supply opening (90) to introduce the fluid under pressure into the manifold (20),
dividing the flow of the fluid to the left and right and through the plurality of passages (86, 86a, 86b) to disperse the fluid under pressure from the manifold throughout the bag, and
draining the fluid and waste materials from the bottom drain opening (30) in the bag (10).

## Patentansprüche

1. Reinigungssystem für einen Kolostomiebeutel, welches enthält:
einen Kolostomiebeutel (10) mit einer unteren Abflussöffnung (30), einer Stomaöffnung (12) und einer Wasserzuführöffnung (90) im oberen Bereich des Beutels, wobei der Beutel von zwei ähnlich geformten Seiten gebildet wird, welche von einer ersten Heißsiegellinie (80a) zusammengehalten werden, die um den Umfang der Seiten herum und ihm benachbart verläuft, um den Beutel zu bilden,
eine heißversiegelte Scheidewand (80), die mehrere zweite heißversiegelte Abschnitte enthält und zwischen den Seiten entlang des oberen Randes und beabstandet vom oberen Rand des Beutels ausgebildet ist, um den Beutel in einen oberen Verteiler (20) und eine untere Kammer zu unterteilen, wobei die Stomaöffnung und die untere Abflussöffnung in der unteren Kammer vorgesehen sind und die Zuführöffnung in den oberen Verteiler mündet, wobei die mehreren heißversiegelten Abschnitte mehrere Durchlässe (86, 86a, 86b) definieren, um Verbindungen zwischen dem oberen Verteiler und der unteren Kammer herzustellen, und
ein System zum Zuführen eines Fluids unter Druck zu der Zuführöffnung und in den Verteiler, wobei die zweiten heißversiegelten Abschnitte derart gestaltet sind, dass Fluid in mehreren Richtungen durch die mehreren Durchlässe hindurch aus dem oberen Verteiler in die untere Kammer gespritzt wird, wenn Fluid unter Druck der Zuführöffnung zugeführt wird.

2. System (10) nach Anspruch 1, wobei der Verteiler (20) durch eine Heißsiegellinie (80) gebildet wird, die vom oberen Rand des Beutels beabstandet ist und mehrere Löcher (86) aufweist, die in der Heißsiegellinie (80) ausgebildet sind.

3. System (10) nach Anspruch 1, wobei die zweiten heißversiegelten Abschnitte von Punktschweißungen (86) gebildet werden.

4. System (10) nach Anspruch 3, wobei
die mehreren Punktschweißungen (86) in ihrem Abstand vom oberen Rand des Beutels (10) versetzt angeordnet sind, oder
wobei die mehreren Punktschweißungen (86) Punktschweißungen enthalten, die dem oberen Rand des Beutels (10) benachbart positioniert sind, oder
wobei die mehreren Punktschweißungen (86) Punktschweißungen enthalten, die einander benachbart sind, oder
wobei der Durchmesser der Punktschweißungen (86) variabel ist.

5. System (10) nach Anspruch 1, welches ferner einen in dem System zum Zuführen von Fluid unter Druck vorgesehenen Behälter (100) mit einem Aktuator (102) enthält, um selektiv ein Behandlungsmaterial in das unter Druck stehende Fluid einzuleiten.

6. System (10) nach Anspruch 1, wobei der Verteiler (20) ein Trennelement (94) enthält, das dazu eingerichtet ist, Fluid von dem Mittelabschnitt des Verteilers (20) weg zu leiten.

7. System (10) nach Anspruch 1, welches Mittel zum Ablassen von angesammeltem Gas über ein geruchshemmendes Filter (112) aufweist.

8. System (10) nach Anspruch 4, wobei die Anzahl der Durchlässe zwischen 14 und 24 und ihre Abmessung zwischen 0,127 und 0,305 cm (0,05 und 0,12 Inch) beträgt

9. System (10) nach Anspruch 5, welches ein Sicherungssystem (100, 102) zum Umfalten des Beutels (10) auf sich selbst und Halten des Beutels (10) in einer Position, in der er ungefähr halb so lang wie ein Beutel der vollen Länge ist, aufweist.

10. Verfahren zum Reinigen des Kolostomiebeutels (10) eines Reinigungssystems nach einem der Ansprüche 1 bis 9, welches die folgenden Schritte enthält:
selektives Anschließen eines Fluids unter Druck über das Zuführsystem an die Zuführöffnung (90), um das Fluid unter Druck in den Verteiler (20) einzuleiten,
Aufteilen des Stroms des Fluids nach links und rechts und durch die mehreren Durchlässe (86, 86a, 86b) hindurch, um das Fluid unter Druck aus dem Verteiler im gesamten Beutel zu verteilen, und
Ablassen des Fluids und von Abfallstoffen aus der unteren Abflussöffnung (30) im Beutel (10).

## Revendications

1. Système de nettoyage de sac de colostomie comprenant :
un sac de colostomie (10) ayant une ouverture inférieure d'écoulement (30), une ouverture de stomie (12) et une ouverture d'alimentation en eau (90) au sommet du sac, ledit sac étant formé de deux côtés formés de manière similaire qui sont maintenus ensemble par une première ligne scellée à la chaleur (80a) autour et adjacente au périmètre des côtés pour former le sac, une cloison scellée à la chaleur (80) comprenant une pluralité de secondes parties scellées à la chaleur formées entre les côtés le long et espacées de la périphérie supérieure du sac pour diviser le sac en un collecteur supérieur (20) et en une chambre inférieure, l'ouverture de stomie et l'ouverture inférieure d'écoulement étant disposées dans la chambre inférieure, et l'ouverture d'alimentation se connectant dans le collecteur supérieur, la pluralité de parties scellées à la chaleur définissant une pluralité de passages (86, 86a, 86b) pour connexion entre le collecteur supérieur et la chambre inférieure, et
un système pour fournir un fluide sous pression à l'ouverture d'alimentation et dans le collecteur,
les secondes parties scellées à la chaleur étant configurées de sorte que du fluide est pulvérisé dans des directions multiples à travers la pluralité de passages depuis le collecteur supérieur jusqu'à la chambre inférieure quand du fluide sous la pression est fourni à l'ouverture d'alimentation.

2. Système (10) selon la revendication 1, ledit collecteur (20) étant formé par une ligne scellée à la chaleur (80) espacée du bord supérieur du sac et ayant une pluralité de trous (86) formés dans la ligne scellée à la chaleur (80).

3. Système (10) selon la revendication 1, lesdites secondes parties scellées à la chaleur étant formées par des points de soudure (86).

4. Système (10) selon la revendication 3, dans lequel
la pluralité de points de soudure (86) est échelonnée dans son espacement à partir du bord supérieur du sac (10), ou
dans lequel la pluralité de points de soudure (86) comprend des points de soudure positionnés adjacents au bord supérieur du sac (10), ou
dans lequel la pluralité de points de soudure (86) comprend des points de soudure adjacents les uns aux autres, ou
dans lequel le diamètre des points de soudure (86) est variable.

5. Système (10) selon la revendication 1, comprenant en outre un puisard (100) prévu dans le système pour fournir du fluide sous pression avec un dispositif d'actionnement (102) pour introduire de manière sélective une matière de traitement dans le fluide sous pression.

6. Système (10) selon la revendication 1, dans lequel ledit collecteur (20) contient un séparateur (94) conçu pour diriger un écoulement de fluide à l'écart de la partie de centre du collecteur (20).

7. Système (10) selon la revendication 1, qui inclut un moyen pour décharger du gaz accumulé à travers un filtre de lutte contre les odeurs (112).

8. Système (10) selon la revendication 4, dans lequel les passages sont au nombre de 14 à 24 et ont une dimension de 0,127 et 0,305 cm respectivement (0,05 et 0,12 pouce).

9. Système (10) selon la revendication 5, qui inclut un système de fixation (100, 102) pour plier le sac (10) sur lui-même et maintenir le sac (10) dans une position où il est approximativement à la moitié de la longueur du sac de pleine longueur.

10. Procédé de nettoyage du sac de colostomie (10) d'un système de nettoyage selon n'importe laquelle des revendications 1 à 9 comprenant les étapes de :
connexion sélective d'un fluide sous pression par le système d'alimentation à l'ouverture d'alimentation (90) pour introduire le fluide sous pression dans le collecteur (20),
séparation du flux du fluide vers la gauche et la droite et à travers la pluralité de passages (86, 86a, 86b) pour disperser le fluide sous pression depuis le collecteur partout dans le sac et
drainage du fluide et des déchets de matières à partir de l'ouverture inférieure d'écoulement (30) dans le sac (10).
